# EUROPEAN PATENT SPECIFICATION

(11) **EP 0 959 927 B1**
(45) Date of publication and mention of the grant of the patent: **20.08.2003**
(21) Application number: 98902329.6
(22) Date of filing: 26.01.1998
(51) Int. Cl.: A61M 16/04, A61F 2/20

(54) **BREATHING PROTECTOR**
ATEMSCHUTZAUSRÜSTUNG
PROTECTEUR RESPIRATOIRE

(30) Priority: 30.01.1997 SE 9700282
(43) Date of publication of application: 01.12.1999
(73) Proprietor: ATOS MEDICAL AB, 242 22 Hörby (SE)
(72) Inventor: LAMBERT, Hans, S-113 51 Stockholm (SE)
(74) Representative: Ström, Tore
(86) International application number: SE9800108
(87) International publication number: WO98033543

(56) References cited:
- WO-A-93/08860
- WO-A-95/17138
- SE-B- 348 643
- SE-B- 385 767
- SE-B- 466 990

## Description

The present invention relates to a breathing protector of the type set forth in the preamble to claim 1.

A person with a stoma in the throat breaths through it fully or partially.

It is known to have such patients wear a protector above the stoma which enables inhaled air to be moistened and heated. An example of such a protector is described in Swedish patent 385 767.

Such a device functions well as a moisturizing device, but it is disadvantageous in that the opening through which the air enters and exits can easily be covered by a shirt, scarf or the like. When this happens, the flow of air through the stoma is obstructed, and this can cause difficulty in breathing.

A device that attempts to alleviate this problem is described in Swedish patent application 9304273-7. This device consists of a movable cover plate that can be pressed down against the surface of a heat-moisture exchanger, thereby shutting off the flow of air, whereupon the air passes through a voice valve located between the oesaphophagus and windpipe of the user. When the valve is not pressed in, air can flow out through the device at right angles to the longitudinal direction of the stoma. The device can thus be worn beneath, for example, a shirt without obstructing the flow of air to and from the user. However, it has the disadvantage of being relatively complicated (it contains a spring-loaded cover and a separate heat-moisture exchanger element, and it has a rigid structure). It is also relatively expensive to manufacture because of its complexity.

In addition to being complicated, the device is relatively long axially due to the fact that the elements which permit air to flow radially are located axially outside the heat-moisture exchanger body.

There are similar devices in which the breathing protector, at its outer axial end surface, is provided with an element that deflects the air flowing axially through the heat-moisture exchanger body to a radial out-flow direction thereby eliminating the clogging problem. These devices are described in Swedish patent applications 348 643, 8800203-5 and 9003720-1. However, these devices also suffer from disadvantages similar to those of the breathing protector described in Swedish patent application 9304273-7.

The purpose of the present invention is, in view of the above, to provide a breathing protector in which the risk of blocking the flow of air is eliminated and which is simultaneously very simple and reliable.

In accordance with this invention, this has been achieved by means of a breathing protector of the type set forth in the preamble to claim 1 which exhibits the special characteristics set forth in this claim.

Thanks to the fact that the actual moisture-heat exchanger body is arranged so that its outlet is in a non-axial direction, air can flow through it even if a shirt or the like covers its outer end, and this is accomplished without having to increase its overall built-in length.

In the present patent application, the outlet and inlet are defined relative to the direction in which exhaled air flows. For inhaling, the conditions are reversed of course so that the outlet serves as an inlet and vice versa.

The outlet from the heat-moisture exchanger body is appropriately arranged so that at least one part of it is in a direction which, for the most part, is fully parallel with the skin adjacent to the stoma, thereby realizing the inventive concept.

In a preferred embodiment, the entire outlet is in a lateral direction, thus making certain that none of the flowing air will be affected by any clothing that comes into contact with the breathing protector.

Therewith, it is preferred that this be accomplished by means of a plate arranged on the outer end of the heat-moisture exchanger body, a simple and purposeful way of doing so. The plate also supports the heat-moisture exchanger body and prevents it from being contaminated by clothing that contacts the outside of the body.

Appropriately, the outlet is fully open around its entire periphery, thereby providing maximum usage of the available space and permitting the design of the breathing protector to be simplified as much as possible, thus making it simple and inexpensive to manufacture and simple for the patient to handle.

In certain cases, it may be appropriate to arrange a casing that surrounds the heat-moisture exchanger body, thereby providing an alternative to the embodiment described immediately above. Here, an opening is made in the casing for the laterally directed outlet. The heat-moisture exchanger body will thus be better protected against external effects, although this is disadvantageous in that the flow-through area of the outlet is reduced, in comparison with the variant described immediately above.

In another preferred embodiment, the heat-moisture exchanger body is connected in such a way that it can be removed at an attachment device applied to the skin of the user. This is advantageous in that the heat-moisture exchanger body can be replaced without loosening the connection to the skin of the user. This makes it considerably more comfortable to use since loosening this connection can cause irritation and pain, especially because it is in the form of an adhesive film.

For this embodiment, it is preferred that the disconnectable joint between the heat-moisture exchanger body and the attachment device comprise an adhesive joint. This provides a very simple design, as compared with using some form of locking or snap-in device, thereby reducing manufacturing costs and making it very easy to replace the heat-moisture exchanger body.

In addition, such an embodiment will eliminate the need for any attachment element that connects the heat-moisture exchanger body to the attachment device by having the heat-moisture exchanger body adhere directly to the attachment device. Here, one side of the adhesive joint comprises a surface of the heat-moisture exchanger body itself.

In accordance with another preferred embodiment, the heat-moisture exchanger body is provided with an open structure. In this concept, the flow-paths through the open structure are oriented in all directions just as, for example, in wadding or the pores in foamed plastic. To provide a laterally-directed outlet, some embodiments will, to be sure, require a up to a 90° deflection of the air flow inside the heat-moisture exchanger body. The structure set forth for this provides a simple and effective implementation.

Another preferred embodiment of the invented breathing protector is equipped with a valve that can shut off the flow of air through the heat-moisture exchanger body, a so-called voice valve.

For the preferred embodiment into which a valve is integrated, the heat-moisture exchanger body comprises an elastic material. Valve action can thus be accomplished easily by compressing the elastic material axially.

For a preferred variant of the embodiment mentioned immediately above, the heat-moisture exchanger itself performs the valve function when its flow paths are pinched together when it is compressed, thereby throttling or shutting off the flow.

In general, the valve device can consist of a flange set at an angle, preferably a right angle, to the plane of contact, said flange being appropriately attached at the attachment device used for the breathing protector. Through interaction with the heat-moisture exchanger body which is compressible because of its elasticity, the voice valve is integrated in such a way that it does not increase the axial length of the breathing protector while increasing its complexity only slightly.

It is thus practical to position the flange in an axial hole made in the heat-moisture exchanger body, thereby creating a preferred embodiment of this alternative. The preferred embodiments of the invention described above and others are set forth in the sub-claims that are dependent on claim 1.

The invention is explained more fully in the detailed description of its preferred embodiments which follows, with reference to the attached drawings of which:
Fig. 1 shows an axial section taken through the first embodiment of the breathing protector in accordance with the invention.
Figs. 2-4 show sections, corresponding to that shown in Fig. 1, of the second, third and fourth embodiments of the invention.
Fig. 5 shows the fifth embodiment of the invention, partially in an axial cross-section and partially in a side view.
Fig. 6 shows a section corresponding to that shown in Fig. 1 of a sixth embodiment of the invention.

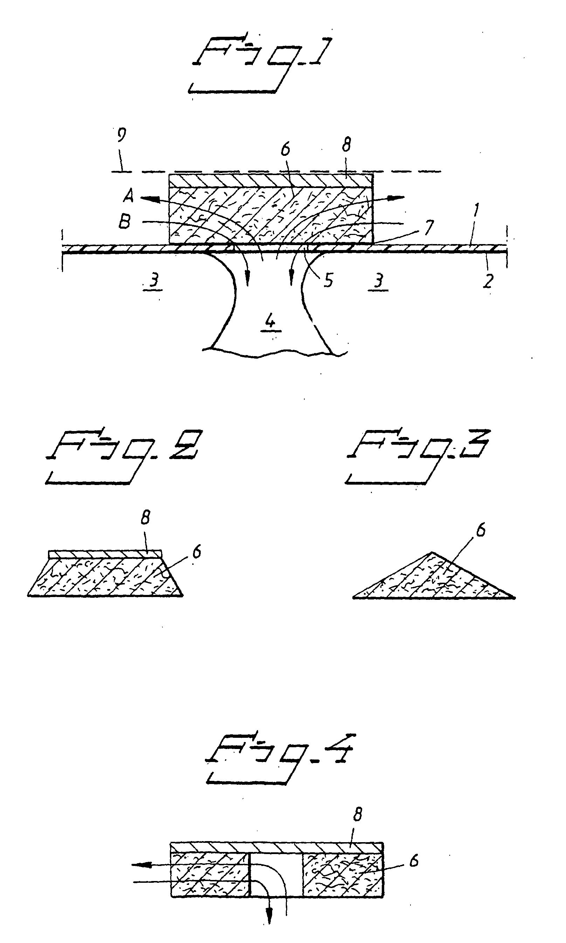

In the embodiment of the breathing protector shown in Fig. 1, it is provided with attachment film 1 having an adhesive layer 2 on its inside, i.e. adhesive tape. The film adheres to skin sections 3 which are located around stoma 4 in the windpipe of the person, and it is provided with through-opening 5 located directly opposite stoma 4.

The breathing protector serves as the main component in a largely circular-cylindrical heat-moisture exchanger body which has a layer 7 of adhesive on the side that is intended to face the skin. The adhesive layer is located on a circular surface on the radial outer part of the aforesaid side, and an uncoated area is left at the centre which is intended to be located directly opposite stoma 4 and opening 5. By means of adhesive layer 7, the heat-moisture exchanger body is attached to the outside of attachment film 1, quite simply by pressing it into contact with this film. It should be understood, of course, that before the heat-moisture exchanger body is used, it is provided with a protective film that covers adhesive joiner 7 and which is removed before the adhesive is applied. At the outer end surface of heat-moisture exchanger body 6, a cover plate 8 is attached.

Arrows A and B illustrate the air-flow path through the heat-moisture exchanger body for exhaling and inhaling respectively. As shown, the air flow is deflected at a right angle to the axial flow through the opening at 5 wherewith, in accordance with the terminology set forth for this application, the part of the heat exchanger that faces this opening is defined as the inlet opening (i.e. relative to the exhaling direction of flow). This deflection is to a flow that is largely radial at the shell surface of the heat-moisture exchanger body, which is defined as the outlet. As shown in the figure, an item of clothing (symbolized by broken line 9) which contacts the outside of the breathing protector will not in any way prevent flow through the heat-moisture exchanger body.

The material in the heat-moisture exchanger body is appropriately soft or elastic so that the breathing protector readily follows the contours of the skin without chafing and without leaks occurring between the tape and skin. The material can comprise, for example, paper, foamed plastic, wadding made of different fibres or combinations of these. It can also be impregnated with a moisture absorbing substance. It is advantageous if the pores in the material do not have any special direction since this makes it easy to have the breathing air pass through the material in a number of directions, thereby achieving deflection.

Cover plate 8 can be made of hard or soft material, but a soft elastic plastic is preferred. The breathing protector can, alternatively, be designed without such a cover plate.

The breathing protector illustrated in Fig. 1 comprises, from many aspects, an optimally designed variant, whose biggest advantages are ease of manufacturing and handling.

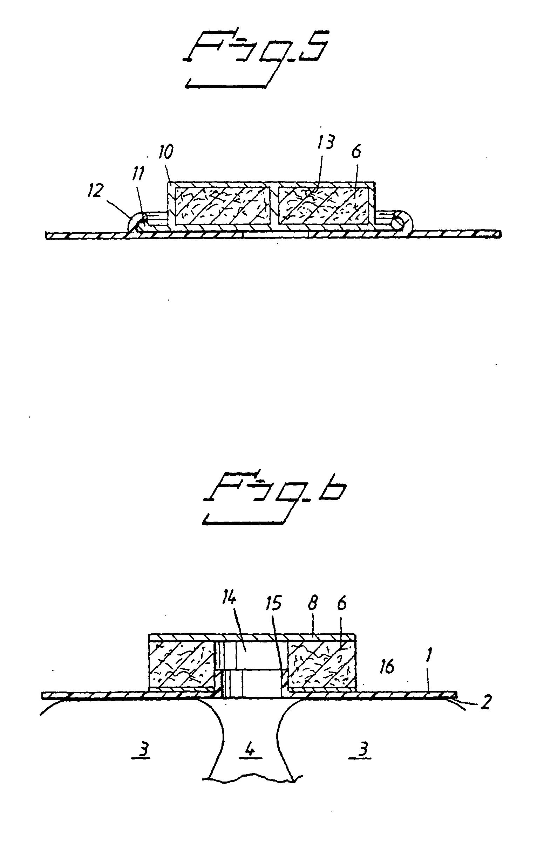

The heat-moisture exchanger body can be shaped in a way that differs in different ways from what is shown. It can, for example, be a truncated cone as shown in Fig. 2, a full cone as shown in Fig. 3 or a cylinder with a hole through it as shown in Fig. 4, and it does not of course have to be circular. For the embodiment shown in Fig. 4, the flow through the heat-moisture exchanger body material can proceed in one direction, and in such case this flow will not be deflected. This provides greater freedom in selecting the structure of the material used in the body.

If a more protected heat-moisture exchanger body is desired by sacrificing a certain amount of simplicity and reducing to some extent the outlet area, it can be housed in casing 10 as shown in Fig. 5. Here, as an alternative to having the heat-moisture exchanger body attached directly to attachment film 1, 2, the heat-moisture exchanger body can adhere to film 1 via casing 10. Casing 10 can therewith either adhere to film 1 or, as shown in the example in the figure, it can be provided with a locking device in, for example, the form of a snap-in flange 11 on casing 10 designed to interact with a snap-in collar 12 on film 1.

Casing 10 is provided with radially oriented openings 13 around its periphery which face heat-moisture exchanger body 6. Openings 13 are relatively large so that the casing, in this area, resembles a cage.

Fig. 6 shows an embodiment in which the breathing protector also incorporates a voice valve. It is therewith an important embodiment of the invention since it was possible to integrate the voice valve with the breathing protector without the breathing protector becoming significantly more complicated. For the sake of clarity, the figure illustrates the attachment device as an axial section taken through it, while the remainder of the breathing protector is shown as a side view. In this case, the heat-moisture exchanger body, just as in the embodiment shown in Fig. 4, is designed as a cylinder with a hole through it through which exhaled air flows axially from stoma 4 into cavity 14 inside heat-moisture exchanger body 6 through which it then flows largely radially. The heat-moisture exchanger body is, at its outer end, covered by plate 8. Film 1 is provided with an outwardly oriented axial flange 15 that surrounds centre opening 5 in the film. In the illustrated embodiment, the heat-moisture exchanger body is made of a relatively soft elastic material.

The valve is closed by, for example, exerting finger pressure against the outside of plate 8, therewith pressing together the heat-moisture exchanger body until the inside of the plate comes into contact with the axial outer end of flange 15. This makes it impossible for air to pass through the heat-moisture exchanger body.

Flange 15 can either be attached to plate 8 and oriented inwards or replaced with a circular band that contacts the wall surface of the cavity. Alternatively, flange 15 or, in applicable cases, the circular band can be placed on the outer shell surface of the heat-moisture exchanger body.

Moreover, the embodiment shown in Fig. 6 shows how heat-moisture exchanger body 6 can also be provided with a cover plate 16 for the inwardly turned end surface, said cover plate being attached to attachment film 1 by means of an adhesive substance. A similar cover plate can also be provided on the inside of the heat-moisture exchanger body for the embodiments shown in Figs. 1-5.

## Claims

1. In the throat that communicates with the windpipe, to be applied to the person's throat to cover the stoma and defining a plane of contact with the stoma and the adjacent part of the skin, said breathing protector incorporating a heat-moisture exchanger body (6) having an inlet for communication with the stoma and an outlet, said body having a substantially flat surface facing the throat of the person when the breathing protector is applied, **characterized in that** said surface is dimensioned to extend beyond the stoma covering the adjacent part of the skin and assumes in use a shape that corresponds to said part, and **in that** said outlet includes at least one part the direction of which has at least one component which is parallel with said plane of contact.

2. Breathing protector in accordance with claim 1 in which the said part of the outlet has a direction that is parallel with the said surface.

3. Breathing protector in accordance with claim 1 or 2 in which the heat-moisture exchanger body (6) has a circular-cylindrical shape and in which the said part of the outlet has a radial orientation.

4. Breathing protector in accordance with any of claims 1-3 in which the entire outlet has the said orientation.

5. Breathing protector in accordance with any of claims 1-4 in which the heat-moisture exchanger body (6) has an end plane that is turned away from said surface, and in which the end plane is covered by a plate.

6. Breathing protector in accordance with any of claims 1-5 in which the said outlet or a part of the said outlet is completely open.

7. Breathing protector in accordance with any of claims 1-5 in which the heat-moisture exchanger body is housed inside a casing (10) with openings (13) for the said outlet or a part of the said outlet.

8. Breathing protector in accordance with any of claims 1-7 incorporating an attachment device used to attach the breathing protector to the skin of the person, wherein said attachment device includes an attachment film (1, 2).

9. Breathing protector in accordance with claim 8 in which the heat-moisture exchanger body (6) is removably attached to said attachment film (1, 2).

10. Breathing protector in accordance with claim 9 in which the heat-moisture exchanger body (6) is removably attached by means of an adhesive (7) to said attachment device.

11. Breathing protector in accordance with claim 10 in which the adhesive (7) joins the heat-moisture exchange body (6) to the attachment film (1, 2).

12. Breathing protector in accordance with any of claims 1-11 in which the heat-moisture exchanger body (6) has an open structure in which the flow paths are not oriented in any special direction.

13. Breathing protector in accordance with any of claims 1-12 in which said inlet is substantially at right angles to the said surface.

14. Breathing protector in accordance with any of claims 1-13 provided with a valve device (15) arranged so that it can shut off or throttle air flow through the heat-moisture exchanger body (6).

15. Breathing protector in accordance with claim 1-14 in which the heat-moisture exchanger body (6) is elastic in at least one direction perpendicular to the said surface, the valve device (15) being arranged to shut off or throttle air flow when the heat-moisture exchanger body is pressed together in the said direction.

16. Breathing protector in accordance with claim 15 in which the valve device comprises a flange (15) with at least one directional component at right angles to said surface.

17. Breathing protector in accordance with claim 15 in which the flange is largely perpendicular to said surface.

18. Breathing protector in accordance with claim 16 or 17 in which the heat-moisture exchanger body (6) is compressible to a thickness which substantially corresponds to the extent of the flange (15) in the said direction.

19. Breathing protector in accordance with any of claims 16-18 in which the flange (15) is in contact with either the outlet or the inlet of the heat-moisture exchanger body.

20. Breathing protector in accordance with claims 16-17 in which the flange is attached to the attachment device (1, 2).

21. Breathing protector in accordance with any of claims 17-20 in which the heat-moisture exchanger body (6) has a through-hole (14) that is largely perpendicular to said surface, the flange (15) being located in said hole (14).

22. Breathing device in accordance with claim 15 in which the flow paths existing in the heat-moisture exchanger body (6) when it is in the said compressed state are pinched together, thereby forming the said valve device.

## Patentansprüche

1. Atemschutzausrüstung für eine laryngectomierte oder tracheotomoierte Person mit einem Stoma (4) in seinem Hals, das mit der Luftröhre in Verbindung steht, an der Kehle der Person zur Abdeckung des Stoma anbringbar ist und eine Berührungsebene mit der Stoma und dem anliegenden Teil der Haut bildet, wobei die Atemschutzausrüstung einen Wärme-Feuchtigkeitstauscherkörper (6) mit einem Einlass zur Verbindung mit der Stoma und eine Auslaßöffnung aufweist, wobei der Körper eine im wesentlichen flache Oberfläche aufweist, die der Kehle der Person zugewandt ist, wenn die Atemschutzausrüstung eingesetzt wird, **dadurch gekennzeichnet, daß** die Oberfläche derart dimensioniert ist, daß sie sich über das Stoma hinaus erstreckt und den anliegenden Teil der Haut abdeckt und während der Benutzung eine Form annimmt, die mit dem genannten Teil korrespondiert, und daß die Auslaßöffnung zumindest einen Teil aufweist, der zumindest eine Richtungskomponente aufweist, die parallel zur genannten Berührungsebene ist.

2. Atemschutzausrüstung nach Anspruch 1 in welcher der genannte Teil des Auslaßkanals eine Richtung aufweist, die parallel zu der genannten Oberfläche ist.

3. Atemschutzausrüstung nach Anspruch 1 oder 2, in welcher der Wärme-Feuchtigkeitstauscherkörper (6) eine kreiszylindrische Form aufweist und in welcher der genannte Teil der Ausla0öffnung eine radiale Orientierung aufweist.

4. Atemschutzausrüstung nach einem der Ansprüche 1 bis 3, in welcher die gesamte Ausla0öffnung die genannte Orientierung aufweist.

5. Atemschutzausrüstung nach einem der Ansprüche 1 bis 4, in welcher der Wärme-Feuchtigkeitstauscherkörper (6) eine Endebene aufweist, die von der genannten Oberfläche weggebogen ist und in der die Endebene durch eine Platte abgedeckt ist.

6. Atemschutzausrüstung nach einem der Ansprüche 1 bis 5, in welcher die genannte Auslaßöffnung oder ein Teil der genannten Auslaßöffnung komplett geöffnet ist.

7. Atemschutzausrüstung nach einem der Ansprüche 1 bis 5, in welcher der Wärme-Feuchtigkeitstauscherkörper innerhalb eines Gehäuses (10) mit Öffnungen (13) für die genannte Auslaßöffnung oder einen Teil der genannten Auslaßöffnung untergebracht ist.

8. Atemschutzausrüstung nach einem der Ansprüche 1 bis 7, mit einer Befestigungsvorrichtung zur Befestigung der Atemschutzausrüstung an der Haut der Person, wobei die genannte Befestigungsvorrichtung eine Befestigungsfolie (1, 2) aufweist.

9. Atemschutzausrüstung nach Anspruch 8, in welcher der Wärme-Feuchtigkeitstauscherkörper (6) entfernbar an der Befestigungsfolie (1, 2) befestigt ist.

10. Atemschutzausrüstung nach Anspruch 9, in welcher der Wärme-Feuchtigkeitstauscherkörper (6) entfernbar mittels eines Klebstoffs (7) an der genannten Befestigungsvorrichtung befestigt ist.

11. Atemschutzausrüstung nach Anspruch 10, in welcher der Klebstoff (7) den Wärme-Feuchtigkeitstauscherkörper (6) mit der Befestigungsfolie (1, 2) verbindet.

12. Atemschutzausrüstung nach einem der Ansprüche 1 bis 11, in welcher der Wärme-Feuchtigkeitstauscherkörper (6) eine offene Struktur aufweist, in der die Strömungswege nicht in einer bestimmten Richtung ausgerichtet sind.

13. Atemschutzausrüstung nach einem der Ansprüche 1 bis 12, in welcher der genannte Einlaß im wesentlichen im rechten Winkel zur genannten Oberfläche angeordnet ist.

14. Atemschutzausrüstung nach einem der Ansprüche 1 bis 13, die mit einer Ventilvorrichtung (15) versehen ist, die derart angeordnet ist, daß diese die Luftströmung durch den Wärme-Feuchtigkeitstauscherkörper (6) absperren oder drosseln kann.

15. Atemschutzausrüstung nach einem der Ansprüche 1 bis 14, in welcher der Wärme-Feuchtigkeitstauscherkörper (6) in zumindest einer senkrechten Richtung zur genannten Oberfläche elastisch ist, wobei die Ventilvorrichtung derart angeordnet ist, daß die Luftströmung abgesperrt oder gedrosselt wird, wenn der Wärme-Feuchtigkeitstauscherkörper in der genannten Richtung zusammengedrückt wird.

16. Atemschutzausrüstung nach Anspruch 15, in welcher die Ventilvorrichtung einen Flansch (15) mit zumindest einer im rechten Winkel zur genannten Oberfläche ausgerichteten Komponente aufweist.

17. Atemschutzausrüstung nach Anspruch 15, in welcher der Flansch weitestgehend senkrecht zur genannten Oberfläche ist.

18. Atemschutzausrüstung nach Anspruch 16 oder 17, in welcher der Wärme-Feuchtigkeitstauscherkörper (6) auf eine Dicke zusammendrückbar ist, die im wesentlichen mit der Ausdehnung des Flansches (15) in der genannten Richtung korrespondiert.

19. Atemschutzausrüstung nach einem der Ansprüche 16 bis 18, in welcher der Flansch (15) entweder mit der Auslaßöffnung oder dem Einlaß des Wärme-Feuchtigkeitstauscherkörpers in Berührung steht.

20. Atemschutzausrüstung nach Anspruch 16 oder 17, in welcher der Flansch an der Befestigungsvorrichtung befestigt ist.

21. Atemschutzausrüstung nach einem der Ansprüche 17 bis 20, in welcher der Wärme-Feuchtigkeitstauscherkörper (6) ein Durchgangsloch (14) aufweist, das weitestgehend senkrecht zur genannten Oberfläche ist, wobei der Flansch (15) in dem Loch (14) angeordnet ist.

22. Atemvorrichtung nach Anspruch 15, in welcher die Strömungswege in dem Wärme-Feuchtigkeitstauscherkörper (6) existieren, wenn es in dem genannten zusammengedrückten Zustand zusammengequetscht ist, damit die genannte Ventilvorrichtung ausgebildet wird.

## Revendications

1. Protecteur respiratoire pour une personne laryngectomisée ou trachéotomisée ayant un orifice de trachéostomie (4) dans la gorge qui communique avec la trachée, à appliquer sur la gorge de la personne pour couvrir l'orifice de trachéostomie et définissant un plan de contact avec l'orifice de trachéostomie et la partie adjacente de la peau, ledit protecteur respiratoire comportant un corps échangeur de chaleur et d'humidité (6) ayant un orifice d'entrée pour la communication avec l'orifice de trachéostomie et un orifice de sortie, ledit corps ayant une surface sensiblement plate faisant face à la gorge de la personne lorsque le protecteur respiratoire est appliqué, **caractérisé en ce que** ladite surface est dimensionnée pour s'étendre au-delà de l'orifice de trachéostomie couvrant la partie adjacente de la peau et prend à l'usage une forme qui correspond à ladite partie, et **en ce que** ledit orifice de sortie comprend au moins une partie dont la direction a au moins une composante qui est parallèle au dit plan de contact.

2. Protecteur respiratoire selon la revendication 1, dans lequel ladite partie de l'orifice de sortie a une direction qui est parallèle à ladite surface.

3. Protecteur respiratoire selon la revendication 1 ou 2, dans lequel le corps échangeur de chaleur et d'humidité (6) a une forme circulaire-cylindrique et dans lequel ladite partie de l'orifice de sortie a une orientation radiale.

4. Protecteur respiratoire selon l'une quelconque des revendications 1 à 3, dans lequel la totalité de l'orifice de sortie a ladite orientation.

5. Protecteur respiratoire selon l'une quelconque des revendications 1 à 4, dans lequel le corps échangeur de chaleur et d'humidité (6) a un plan d'extrémité qui est détourné de ladite surface, et dans lequel le plan d'extrémité est couvert par une plaque.

6. Protecteur respiratoire selon l'une quelconque des revendications 1 à 5, dans lequel ledit orifice de sortie ou une partie dudit orifice de sortie est complètement ouvert.

7. Protecteur respiratoire selon l'une quelconque des revendications 1 à 5, dans lequel le corps échangeur de chaleur et d'humidité est logé à l'intérieur d'un boîtier (10) avec des ouvertures (13) pour ledit orifice de sortie ou une partie dudit orifice de sortie.

8. Protecteur respiratoire selon l'une quelconque des revendications 1 à 7, comportant un dispositif de fixation utilisé pour fixer le protecteur respiratoire à la peau de la personne, dans lequel ledit dispositif de fixation comprend un film de fixation (1, 2).

9. Protecteur respiratoire selon la revendication 8, dans lequel le corps échangeur de chaleur et d'humidité (6) est fixé de façon amovible au dit film de fixation (1, 2).

10. Protecteur respiratoire selon la revendication 9, dans lequel le corps échangeur de chaleur et d'humidité (6) est fixé de façon amovible au moyen d'un adhésif (7) au dit dispositif de fixation.

11. Protecteur respiratoire selon la revendication 10, dans lequel l'adhésif (7) joint le corps échangeur de chaleur et d'humidité (6) au film de fixation (1, 2).

12. Protecteur respiratoire selon l'une quelconque des revendications 1 à 11, dans lequel le corps échangeur de chaleur et d'humidité (6) a une structure ouverte dans laquelle les trajectoires de l'écoulement ne sont dirigées dans aucune direction particulière.

13. Protecteur respiratoire selon l'une quelconque des revendications 1 à 12, dans lequel ledit orifice d'entrée est sensiblement à angle droit avec ladite surface.

14. Protecteur respiratoire selon l'une quelconque des revendications 1 à 13, muni d'un dispositif de clapet (15) disposé de telle sorte qu'il peut couper ou réduire l'écoulement de l'air à travers le corps échangeur de chaleur et d'humidité (6).

15. Protecteur respiratoire selon l'une quelconque des revendications 1 à 14, dans lequel le corps échangeur de chaleur et d'humidité (6) est élastique dans au moins une direction perpendiculaire à ladite surface, le dispositif de clapet (15) étant conçu pour couper ou réduire l'écoulement de l'air lorsque le corps échangeur de chaleur et d'humidité est comprimé dans ladite direction.

16. Protecteur respiratoire selon la revendication 15, dans lequel le dispositif de clapet comprend une bride (15) ayant au moins une composante directionnelle à angle droit avec ladite surface.

17. Protecteur respiratoire selon la revendication 15, dans lequel la bride est dans une large mesure perpendiculaire à ladite surface.

18. Protecteur respiratoire selon la revendication 16 ou 17, dans lequel le corps échangeur de chaleur et d'humidité (6) est compressible à une épaisseur qui correspond sensiblement à la longueur de la bride (15) dans ladite direction.

19. Protecteur respiratoire selon l'une quelconque des revendications 16 à 18, dans lequel la bride (15) est en contact soit avec l'orifice de sortie soit avec l'orifice d'entrée du corps échangeur de chaleur et d'humidité (6).

20. Protecteur respiratoire selon les revendications 16 et 17, dans lequel la bride est fixée au dispositif de fixation (1, 2).

21. Protecteur respiratoire selon l'une quelconque des revendications 17 à 20, dans lequel le corps échangeur de chaleur et d'humidité (6) a un trou qui le traverse (14) qui est dans une large mesure perpendiculaire à ladite surface, la bride (15) étant située dans ledit trou (14).

22. Protecteur respiratoire selon la revendication 15, dans lequel les trajectoires d'écoulement existant dans le corps échangeur de chaleur et d'humidité (6) lorsqu'il est dans ledit état de compression sont pincées, de ce fait formant ledit dispositif de clapet.
